# EUROPEAN PATENT APPLICATION

(11) **EP 2 085 402 A1**
(43) Date of publication of application: **05.08.2009**
(21) Application number: 09153345.5
(22) Date of filing: 14.04.2004
(51) Int. Cl.: C07F 9/09, C07F 9/10, C07F 9/12, C07F 9/117, A61K 9/08, A61K 31/661, A61K 31/6615, A61K 31/683, A61K 31/685, A61P 9/00, A61P 23/00

(54) **Phosphates of secondary alcohols**

(30) Priority: 15.04.2003 AU 2003901812
(62) Divisional of application: 04727180.4
(71) Applicant: Vital Health Sciences Pty Ltd., Melbourne, VIC 3000 (AU)
(72) Inventor: West, Simon Michael, Williamstown, VIC 3016 (AU); Kannar, David, Belgrave South, VIC 3160 (AU)
(74) Representative: Vossius & Partner

(57) **Abstract**

The present invention relates to a phosphate derivative of atorvastatin or a derivative thereof. A method for phosphorylating atorvastatin or a derivative thereof comprising reacting the compound with P₄O₁₀ in the presence of an alkali metal salt of a fatty acid is also disclosed. The claimed phosphate derivative can be used to lower patient serum cholesterol levels or treat depression.

## Description

### Field of the invention

The invention relates to phosphate derivatives of compounds having a secondary hydroxy group.

### Background of the invention

In this specification, where a document, act or item of knowledge is referred to or discussed, this reference or discussion is not an admission that the document, act or item of knowledge or any combination thereof was at the priority date part of common general knowledge; or known to be relevant to an attempt to solve any problem with which this specification is concerned.

Whilst the following discussion relates to phosphate and phosphate complex derivatives of venlafaxine, pravastatin and atorvastatin, it will be understood that the invention has applications to other pharmaceuticals having secondary hydroxy groups where improved water solubility, tissue penetration, lymphatic transport or decreased first pass metabolism may be desired. Furthermore, whilst the following discussion emphasizes pharmaceuticals having antidepressant and cholesterol lowering characteristics, it will be understood that the invention is not so limited but includes pharmaceuticals having other characteristics.

### Hypercholesterolaemic drugs - ravastatin and atorvastatin

The association between high total serum cholesterol and an increased risk of cardiovascular disease has been known for decades. The risk of cardiovascular disease increases with increasing levels of serum total cholesterol, increasing levels of serum LDL cholesterol and decreasing levels of serum HDL cholesterol.

Methods of lowering serum cholesterol vary from dietary management through to surgical removal of bowel loops and various pharmaceutical approaches. Although dietary and lifestyle management will always remain first line treatment, pharmaceuticals intervention is often needed to achieve clinically significant reduction in elevated serum cholesterol. The largest therapeutic group to be marketed for reduction of serum cholesterol are competitive inhibitors of HMG-CoA reductase (3-hydroxy-3-methylglutaryl-coenzyme A) or the "stains". These drugs specifically inhibit the liver enzyme HMG-CoA reductase which responsible for converting HMG-CoA to melavonate. This conversion is the rate-limiting step in de-novo synthesis of cholesterol in the body. Approximately 90% of the body's cholesterol is manufactured via this pathway so HMG-CoA reductase inhibitors are very effective agents and reduce serum cholesterol more significantly than previous therapies.

Statins are structural analogues of MMG-CoA reductase and work by inhibiting this enzyme responsible for catalysing the rate-limiting step in the biosynthesis of cholesterol. The first drug in this class - compactin was developed in the early 1980's and was soon followed by lovastatin, atorvastatin, fluvastatin, pravastatin and simvestatin. Production of cholesterol in the liver follows a diurnal pattern, with the majority being produced during the night. For this reason, most statins are administered to a patient at night so that they are active during the period of greatest cholesterol synthesis. Lovastatin and simvestatin are inactive pro-drugs that are hydrolysed in the gastrointestinal tract to active beta-hydroxyl derivatives. Atorvastatin, pravastatin and fluvastatin are active drugs as given.

Pravastatin is rapidly absorbed into the blood after oral administration. Plasma concentrations are proportional to the dose administered and elimination half-life is between 1.5 to 2 hours. Although peak plasma levels are attained after about an hour and a half with absorption ranges from 30 to 50%, absolute bioavailability is only around 17% due to a very high first pass metabolism. Therefore, an improved formulation would decrease the amount lost through first pass metabolism. Pravastatin (CAS 81093-37-0) and its sodium salt (CAS 81131-70-6) are the only forms currently known.

Similarly, atorvastatin is rapidly absorbed with maximal plasma concentrations occurring approximately 2 hours after administration, however the absolute bioavailability is only 14%. The low systemic bioavailability is due to gastrointestinal mucosal clearance and/or high hepatic first pass metabolism. Again, an improved formulation would have an increased absolute bioavailability. Atorvastatin is currently used in its free acid form (CAS 134523-00-5) and its calcium salt (CAS 134523-03-8).

The drugs provide their maximum benefit when they reach the liver. However, absorption and activity of the drug is often impeded by:
(a) liver tissue uptake,
(b) low elimination half life values, and
(c) low levels of accumulation of the drug in the liver.

### Antidepressant - venlafaxine

Depression is one of the most common psychiatric disorders, reported to affect at any given moment up to 5 - 6% of the population. Symptoms of depression are often vague or subtle and manifestation is sometimes unrecognisable both by patients and physicians.

After the introduction of reserpine in the 1950's for treatment of hypertension, it became apparent that one of the drug's side effects was depression. Pharmacologic studies of this effect revealed that reserpine inhibited the storage of amine neurotransmitters seratonin and norepinephrine (adrenalin) in vesicles of the presynaptic nerve endings. It was therefore concluded that depression must involve depletion or decreased function of amine dependant synaptic transmission. This simple syllogism provided what was known as the gamine hypothesis of depression. The first antidepressants introduced were monoamine oxidase inhibitors (MAOI) and shortly after their release tricyclic antidepressants (originally derived from an antihistamine) were launched. Treatment of depression has therefore undergone many changes over time as further discoveries about the biochemistry of depression have been made.

Venlafaxine was approved for use as an antidepressant at the end of 1993. It is an opiate derivative which appears to inhibit re-uptake of noradrenaline, serotonin and dopamine - thus increasing their levels and reducing symptoms of depression. The compound therefore acts to potentiate neurotransmitter activity in the central nervous system (CNS). In theory it combines all the known modes of antidepressant action but has little effect on cholinergic, histaminergic or adrenergic receptors and therefore causes fewer side effects commonly associated with other antidepressants.

The commercially produced drug, venlafaxine hydrochloride, is a racemate. The R-enantiomer is a more potent inhibitor of noradrenaline uptake and the S-enantiomer is a more potent inhibitor of seratonin uptake. Both however are more potent on blocking seratonin uptake than noradrenaline reuptake.

Venlafaxine is well absorbed orally but has a short half-life and is subject to extensive first pass metabolism. The major metabolite of venlafaxine is O-desmethylvenlafaxine which is equally potent as venlafaxine. An extended release formulation has therefore become available to allow once daily dosing. Mean peak plasma concentrations following single 25 to 150 mg doses, range from approximately 33 to 175 ng/ml reached in approximately 2.4 hours. Half-life of venlafaxine is calculated to be 5 hours and O-desmethylvenlafaxine is 11 hour.

Venlafaxine and its metabolites are primarily excreted via the kidneys with approximately 85% being recovered in the urine 48 hours after dosage as unchanged drug, O-desmethylvenlafaxine or conjugated O-desmethylvenlafaxine. Administration with food slightly delays peak plasma concentration but does not influence formation of O-desmetliylvenlafaxine.

Although pravastatin sodium, atorvastatin calcium and venlafaxine hydrochloride are well absorbed, these drugs suffer high first pass metabolism which decreases their absolute bioavailability.

Accordingly there exists a need for improved pharmaceuticals which are less prone to loss through first pass metabolism as compared with pharmaceuticals having secondary alcohol groups of the prior art.

### Summary of the invention

It was found that secondary alcohols are readily phosphorylated in the presence of sodium salts of fatty acids and P₄O₁₀. Previous methods used to phosphorylate these secondary alcohols had the disadvantage of causing a significant degree of dehydration of the secondary alcohol to form a double bond.

According to a first aspect of the invention, there is provided a phosphate derivative of a compound selected from the group consisting of pravastatin and derivatives thereof, atorvastatin and derivatives thereof, venlafaxine and derivatives thereof and mixtures thereof.

In a preferred embodiment the phosphate derivative may be a phosphatide. In another preferred embodiment the phosphate derivative is a complex formed with a complexing agent selected from the group comprising amphoteric surfactants, cationic surfactants, amino acids having nitrogen functional groups and proteins rich in these amino acids.

According to a second aspect of the invention, there is provided a method for preparing a phosphate derivative of a compound having a secondary hydroxy group comprising the step of reacting the compound having a secondary hydroxy group with P₄O₁₀ in the presence of an alkali metal salt of a fatty acid.

The alkali metals are known to persons skilled in the art and include sodium and potassium. Preferably the alkali metal is sodium.

Suitable fatty acids are known to persons skilled in the art and include oleic acid and valeric acid.

Examples of compounds having secondary hydroxy groups which may be used in this method include venlafaxine (CAS 93413-69-5), pravastatin (CAS 81093-37-0) and atorvastatin (CAS 134523-00-5).

In a preferred embodiment, the method further comprises the step of reacting the phosphate derivative of a compound having a secondary hydroxy group with a di or mono acyl glyceride to form a phosphatide derivative of the compound having a secondary hydroxy group.

Where used herein the term "phosphate derivatives" refers to compounds covalently bound by means of an oxygen to the phosphorus atom of a phosphate group. The phosphate derivative may exist in the form of a free phosphate acid, a salt thereof, a di-phosphate ester thereby including two molecules of the compound having a secondary hydroxy group, a mixed ester including two different compounds, a phosphatidyl compound wherein the free phosphate oxygen forms a bond with an alkyl or substituted alkyl group such as a di or mono acyl glyceride or as a complex with a complexing agent.

Suitable completing agents for use in the present invention may be selected from surfactants chosen from the classes including imino compounds, alkyl amino/amido betaines, sultaines, phosphobetaines, phosphitaines, imidazolimum and straight chain mono and dicarboxy ampholytes, quaternary ammonium salts, and cationic alkoxylated mono and di-fatty amines; and amino acids having nitrogen functional groups and proteins rich in these amino acids. Preferred complexing agents N-lauryl imino di-propionate and arginine.

Suitable amino acids having nitrogen functional groups for use in the present invention include glycine, arginine, lysine and histidine. Proteins rich in these amino acids may also be used as complexing agents, for example, casein. These complexing agents are used when the composition needs to be orally ingestible.

The amphoteric surfactants may be ampholytic surfactants, that is, they may exhibit a pronounced isoelectric point within a specific pH range; or zwitterionic surfactants, that is, they are cationic over the entire pH range and do not usually exhibit a pronounced isoelectric point. Examples of these amphoteric surfactants are tertiary substituted amines, such as those according to the following formula:

NR¹R²R³

wherein R¹ is chosen from the group comprising straight or branched chain mixed alkyl radicals from C6 to C22 or carbonyl derivatives thereof.
R² and R³ are independently chosen from the group comprising H, CH₂COOX,
CH₂CHOHCH₂SO₃X, CH₂CHOHCH₂OPO₃X, CH₂CH₂COOX, CH₂COOX,
CH₂CH₂CHOHCH₂SO₃X or CH₂CH₂CHOHCH₂OPO₃X and X is H, Na, K or alkanolamine provided that R² and R³ are not both H.

In addition, when R¹ is RCO then R² may be CH₃ and R³ may be (CH₂CH₂)N(C₂H₄OH)-H₂CHOPO₃ or R² and R³ together may be N(CH₂)₂N(C₂H₄OH)CH₂COO-.

Commercial examples are DERIPHAT sold by Henkel/Cognis, DEHYTON sold by Henkel/Cognis, TEGOBETAM sold by Goldschmidt and MIRANOL sold by Rhone Poulenc.

Cationic surfactant, such as quaternary ammonium compounds, will also form complexes with phosphorylated derivatives of drug hydroxy compounds such as tocopheryl phosphates. Examples of cationic surfactants include the following:
(a) RN⁺(CH₃)₃ Cl⁻
(b) [R₂N⁺CH₃]₂ SO₄²⁻
(c) [RCON(CH₃)CH₂CH₂CH₂N⁺(CH₃)₂C₂H₄OH]₂ SO₄²⁻
(d) Ethomeens: RN[(CH₂CH₂O)ₓ CH₂OH][(CH₂CH₂O)_{y} CH₂OH] wherein x and y are integers from 1 to 50.
wherein R is C8 to C22 straight or branched chain alkyl groups or mixed alkyl groups.

Silicone surfactants including hydrophilic and hydrophobic functionality may also be used, for example, dimethicone PG betaine, amodimethicone or trimethylsilylamodimethicone. For example, ABILE 9950 from Goldschmidt Chemical Co. The hydrophobe can be a C6 to C22 straight -or branched alkyl or mixed alkyl including fluoroalkyl, fluorosilicone and or mixtures thereof. The hydrophilic portion can be an alkali metal, alkaline earth or alkanolamine salts of carboxy alkyl groups or sulfoxy alkyl groups, that is sultaines, phosphitaines or phosphobetaines or mixtures thereof.

Typically, the complex of the phosphate derivative of the compound having a secondary hydroxy group may be made by (1) direct neutralization of the free phosphoric acid ester of the pravastatin, atorvastatin or venlafaxine with the complexing agents or (2) in-situ blending of mixed sodium salts of the phosphate derivatives of the compound having a secondary hydroxy group with the complexing agents.

The phosphate derivatives of compounds having a secondary hydroxy group according to the invention when used in a suitable route of administration (oral, transmucosal, intranasal, transdermal, intravenous or combinations thereof) may provide various benefits including:
(a) improved water solubility eliminating need for dissolution in lipidic vehicles and side effects associated with these compounds;
(b) delivery of the compound primarily to the lymphatic system reducing the extent of first pass metabolism;
(c) increased liver tissue specificity leading to a higher accumulation in liver tissue with a longer elimination half-life;
(d) increased systemic bioavailability following dermal delivery;
(e) potential use as a chronic delivery system because of improved dermal penetration and smoother absorption kinetics leading to a lower side effect profile;
(f) potential use as an enteric coated transfer protein complex;
(g) potential use as an active domain attachment; and
(h) increased bioavailability in the CNS reducing the amount of drug needed for therapeutic efficacy.

The present invention is summarized by the following items:
1. A phosphate derivative of a compound selected from the group consisting of pravastatin and derivatives thereof, atorvastatin and derivatives thereof, venlafaxine and derivatives thereof and mixtures thereof.
2. The phosphate derivative according to item 1 wherein the phosphate derivative is a phosphatide.
3. The phosphate derivative according to item 1 wherein the phosphate derivative is a complex, the complexing agent being selected from the group consisting of amphoteric surfactants, cationic surfactants, amino acids having nitrogen functional groups and proteins rich in these amino acids, and fixtures thereof.
4. The phosphate derivative according to item 3 wherein the complexing agent is selected from the group consisting of glycine, arginine, lysine, histidine and lauryl-imino-dipropionate.
5. A method for phosphorylating a compound having a secondary hydroxy group comprising step (a) reacting the compound having a secondary hydroxy group with P₄O₁₀ in the presence of an alkali metal salt of a fatty acid.
6. The method according to item 5 wherein the compound having a secondary hydroxy group is selected from the group consisting of pravastatin, atorvastatin or venlafaxine.
7. The method according to item 5 wherein the alkali metal salt of a fatty acid is sodium valerate.
8. The method according to item 5 further comprising step (b) reacting the product of step (a) with a di or mono acyl glyceride to form a phosphatide.
9. The method according to item 5 further comprising step (b') reacting the product of step (a) with a complexing agent is selected from the group comprising amphoteric surfactants, cationic surfactants, amino acids having nitrogen functional groups and proteins rich in these amino acids.
10. The method according to item 8 further comprising step (c) reacting the product of step (b) with a complexing agent is selected from the group comprising amphoteric surfactants, cationic surfactants, amino acids having nitrogen functional groups and proteins rich in these amino acids.
11. The method according to either of items 9 or 10 wherein the completing agent is selected from the group consisting of glycine, arginine, lysine, histidine and lauryl-imino-dipropionate
12. A phosphate derivative comprising the reaction product of a compound having a secondary hydroxy group reacted with P₄O₁₀ in the presence of an alkali metal salt of a fatty acid.
13. A phosphate derivative selected from the group consisting of [R-(R*,R*)]-2-(4-fluorophenyl)-β-phosphono-δ-hydroxy-5-(1-methylethyl)-3-phenyl-4-[(phenylamino)carbonyl]-1*H*-pyrrole-1-heptanoic acid, [1S-[1α(βS*,δS*),2α,6α,8β(R*),8aα]]-1,2,6,7,8,8a-hexahydro-β-phosphono-δ,6-dihydroxy-2-metliyl-8-(2-metliyl-1-oxobutoxy)-1-naphthiencheptanoic acid, 1-[-(dimethylamino)-1-(4-methoxyphenyl)ethyl]cyclohexyl dihydrogen phosphate and mixtures thereof.
14. A phosphate derivative selected from then group consisting of 1,2-distearoyl phosphatidyl atorvastatin, 1,2-distearoyl phosphatidyl pravastatin, 1,2-distearayl phosphatidyl venlafaxine and mixtures thereof.
15. A phosphate derivative according to any one of items 1 to 3 or 12 to 14 when administered to a patient to lower patient serum cholesterol levels.
16. A phosphate derivative according to any one of items 1 to 3 or 12 to 14 when administered to a patient to treat depression.

### Examples

The invention will now be further explained and illustrated by reference to the following nonlimiting examples.

### Example 1 - preparation of phosphate derivative of atorvastatin

The free acid of atorvastatin 55.8 g (0.1M) and 37.2 g of sodium valerate (0.3M) were dissolved in 100 ml toluene. 12.6 g (0.05M) of P₄O₁₀ was added and mixed with high shear mixing for one hour slowly raising the temperature to 80°C. 10 ml of water was added and the high sheer mixing continued for a further hour at 60°C. 100 ml of a 0.1M sodium carbonate solution was added and the mixture gently stirred then centrifuged and the process repeated. The toluene phase was recovered and washed with 100 ml of 0.1M hydrochloric acid. The toluene phase was recovered and the toluene and valeric acid removed under vacuum to give the phosphoric ester of atorvastatin ([R-(R*,R*)]-2-(4-fluorophenyl)-β-phosphono-δ-hydroxy-5-(1-methylethyl)-3-phenyl-4-[(phenylamino)earbonyl]-1*H*-pyrrole-1-heptanoic acid).

### Example 2- preparation of phosphate derivative of Pravastatin

The free acid of pravastatin 42.5 g (0.1M) and 37.2 g of sodium valerate (0.3M) were dissolved in 100 ml toluene. 12.6 g (0.05M) of P₄O₂₀ was added and mixed with high shear mixing for one hour slowly raising the temperature to 80°C. 10 ml of water was added and the high sheer mixing continued for a further hour at 60°C. 100 ml of a 0.1M sodium carbonate solution was added and the mixture gently stirred then centrifuged and the process repeated. The toluene phase was recovered and washed with 100 ml of 0.1M hydrochloric acid. The toluene phase was recovered and the toluene and valeric acid removed under vacuum to give the phosphoric ester of pravastatin ([1S-[1α(βS*,δS*),2α,6α,8β(R*),8aα]]-1,2,6,7,8,8a-hexahydro-β-phosphono-δ,6-dihydroxy-2-methyl-8-(2-methyl-1-oxobutoxy)-1-naphthleneheptanoic acid).

### Examples 3- preparation of phosphate derivative of Venlafaxine

The free acid of venlafaxine 27.7 g (0.1M) and 37.2 g of sodium valerate (0.3M) were dissolved in 100 ml toluene. 12.6 g (0.05M) of P₄O₁₀ was added and mixed with high shear mixing for one hour slowly raising the temperature to 80°C. 10 ml of water was added and the high sheer mixing continued for a further hour at 60°C. 100 ml of a 0.1M sodium carbonate solution was added and the mixture gently stirred then centrifuged and the process repeated. The toluene phase was recovered and washed with 100 ml of 0.1M hydrochloric acid. The toluene phase was recovered and the toluene and valeric acid removed under vacuum to give the phosphoric ester of venlafaxine (1-[-(dimathylamino)-1-(4-methoxyphenyl)ethyl]cyclohexyl dihydrogen phosphate).

### Example 4- preparation of phosphatidyl derivative of atorvastatin

The free acid of atorvastatin 55.8g (0.1M) and 37.2g of sodium valerate (0.3M) were dissolved in 100 ml toluene. 12.6 g (0.05M) of P₄O₁₀ was added and mixed with high shear mixing for one hour slowly raising the temperature to 80°C. 1,2-distearoyl glycerol 30 g was added and the high sheer mixing continued for a further hour at 60°C. 100 ml of a 0.5M sodium hydroxide solution was added and the mixture gently stirred then centrifuged and the process repeated. The toluene phase was recovered and washed with 100 ml of 0.1M hydrochloric acid. The toluene phase was recovered and the toluene and valeric acid removed under vacuum to give 1,2-distearoyl phosphatidyl atorvastatin.

Atorvastatin phosphate was recovered from the aqueous phases.

### Example 5 - preparation of phosphatidyl derivative of pravastatin

The free acid of pravastatin 42.5 g (0.1M) and 37.2 g of sodium valerate (0.3M) were dissolved in 100 ml toluene. 12.6 g (0.05M) of P₄O₁₀ was added and mixed with high shear mixing for one hour slowly raising the temperature to 80°C. 1,2-distearoyl glycerol 30 g was added and the high sheer mixing continued for a further hour at 60°C. 100 ml of a 0.5M sodium hydroxide solution was added and the mixture gently stirred then centrifuged and the process repeated. The toluene phase was recovered and washed with 100 ml of 0.1M hydrochloric acid. The toluene phase was recovered and the toluene and valeric acid removed under vacuum to give 1,2-distearoyl phosphatidyl pravastatin.

Pravastatin phosphate was recovered from the aqueous phases.

### Example 6 - preparation of phosphatidyl derivative of venlafaxine

The free acid ofvenlafaxine 27.7 g (0.1M) and 37.2 g of sodium valerate (0.3M) were dissolved in 100 ml toluene. 12.6 g (0.05M) of P₄O₁₀ was added and mixed with high shear mixing for one hour slowly raising the temperature to 80°C. 1,2-distearoyl glycerol 30 g was added and the high sheer mixing continued for a further hour at 60°C. 100 ml of a 0.5M sodium hydroxide solution was added and the mixture gently stirred then centrifuged and the process repeated. The toluene phase was recovered and washed with 100 ml of 0.1M hydrochloric acid. The toluene phase was recovered and the toluene and valeric acid removed under vacuum to give 1,2-distearoyl phosphatidyl venlafaxine.

Venlafaxine phosphate was recovered from the aqueous phases.

### Example 7 - preparation of complex of phosphate derivative of pravastatin

50.45 (0.1M) of the phosphoric acid ester of pravastatin was mixed with 40.4 (0.1M) of lauryl-imino-dipropionate in 200 ml of water (equimolar proportions). The mixture was mixed thoroughly with good agitation. The final pH was adjusted as desired using small amounts of either component. The mixture was then freeze dried to give the solid complex as a powder (32-33% active).

### Example 3 - preparation of complex of phosphate derivative of pravastatin

50.45g (0.1M) of the phosphoric acid ester of pravastatin was mixed with 17.4g (0.1M) of arginine in 200 ml of water (equimolar proportions). The mixture was mixed thoroughly with good agitation. The final pH was adjusted as desired using small amounts of either component. The mixture was then freeze dried to give the solid complex as a powder.

### Example 9 - preparation of complex of phosphatidyl derivative of venlafaxine

88.4 g (0.1M) of venlafaxine phosphatide was mixed with 40.4 g (0.1M) of lauryl-imino-dipropionate in 200 ml of water (equimolar proportions). The mixture was mixed thoroughly with good agitation. The final pH was adjusted as desired using small amounts of either component. The mixture was then freeze dried to give the solid phospatidyl venlafaxine deriphat complex as a powder.

### Example 10 - preparation of complex of phasphatidyl derivative of venlafaxine

88.4 g (0.1M) of Venlafaxine phosphatide was mixed with 17.4 g (0.1M) of arginine in 200 ml of water (equimolar proportions). The mixture was mixed thoroughly with good agitation. The final pH was adjusted as desired using small amounts of either component. The mixture was then freeze dried to give the solid phosphatidyl venlafaxine arginine complex as a powder.

The word 'comprising' and forms of the word 'comprising' as used in this description and in the claims does not limit the invention claimed to exclude any variants or additions.

Modifications and improvements to the invention will be readily apparent to those skilled in the art. Such modifications and improvements are intended to be within the scope of this invention.

## Claims

1. A phosphate derivative of atorvastatin or a derivative thereof.

2. The phosphate derivative according to claim 1 wherein the phosphate derivative is in the form of a free phosphate acid, a salt thereof, a di-phosphate ester thereby including two molecules of atorvastatin or a derivative thereof, a mixed ester including two different compounds, or a phosphatidyl compound wherein the free phosphate oxygen forms a bond with an alkyl or substituted alkyl group, preferably a di- or mono-acyl glyceride.

3. The phosphate derivative according to claim 1 wherein the phosphate derivative is in the form of a complex with a complexing agent.

4. The phosphate derivative according to claim 3 wherein the complexing agent is selected from the group consisting of amphoteric surfactants, cationic surfactants, amino acids having nitrogen functional groups and proteins rich in these amino acids, and mixtures thereof.

5. The phosphate derivative according to claim 3 wherein the complexing agent is glycine, arginine, lysine, histidine or a compound of the following formula:
NR¹R²R³
wherein R¹ is chosen from the group consisting of straight or branched chain mixed alkyl radicals from C6 to C22 and carbonyl derivatives thereof, and
R² and R³ are chosen independently from the group consisting of H, CH₂(CO)OX, CH₂CH(OH)CH₂SO₃X, CH₂CH(OH)CH₂OPO₃X, CH₂CH₂(CO)OX, CH₂(CO)OX, CH₂CH₂CH(OH)CH₂SO₃X and CH₂CH₂CH(OH)CH₂OPO₃X, wherein X is H, Na, K or alkanolamine provided R² and R³ are not both H; or
wherein R¹ is R¹(CO), R² is CH₃ and R³ is (CH₂CH₂)N(CH₁CH₂(OH))CH₂OPO₃ or R² and R³ taken together are N(CH₂)₂N(CH₂CH₂(OHO))CH₂(CO)O-.

6. The phosphate derivative according to claim 1 wherein the complexing agent is selected from arginine and lauryliminodipropionate.

7. A method for phosphorylating atorvastatin or a derivative thereof comprising step (a) reacting the compound with P₄O₁₀ in the presence of an alkali metal salt of a fatty acid.

8. The method according to claim 7 wherein the alkali metal is sodium or potassium.

9. The method according to claim 7 wherein the fatty acid is oleic acid or valeric acid.

10. The method according to claim 7 wherein the alkali metal salt of the fatty acid is sodium valerate.

11. The method according to claim 7 further comprising step (b) reacting the product of step (a) with a di- or mono-acyl glyceride to form a phosphatide.

12. The method according to claim 7 further comprising step (b') reacting the product of step (a) with a complexing agent selected from the group consisting of amphoteric surfactants, cationic surfactants, amino acids having nitrogen functional groups, proteins rich in these amino acids and mixtures thereof.

13. The method according to claim 11 further comprising step (c) reacting the product of step (b) with a complexing agent selected from the group comprising amphoteric surfactants, cationic surfactants, amino acids having nitrogen functional groups, proteins rich in these amino acids, and mixtures thereof.

14. The method according to claim 12 or 13 wherein the complexing agent is selected from the group consisting of glycine, arginine, lysine, histidine or a compound of the following formula:
NR¹R²R³
wherein R¹ is chosen from the group consisting of straight or branched chain mixed alkyl radicals from C6 to C22 and carbonyl derivatives thereof, and
R² and R³ are chosen independently from the group consisting of H, CH₂(CO)OX, CH₂CH(OH)CH₂SO₃X, CH₂CH(OH)CH₂OPO₃X, CH₂CH₂(CO)OX, CH₂(CO)OX, CH₂CH₂CH(OH)CH₂SO₃X and CH₂CH₂CH(OH)CH₂OPO₃X, wherein X is H, Na, K or alkanolamine provided R² and R³ are not both H; or
wherein R¹ is R¹(CO), R² is CH₃ and R³ is (CH₂CH₂)N(CH₂CH₂(OH))CH₂OPO₃ or R² and R³ taken together are N(CH₂)₂N(CH₂CH₂(OH))CH₂(CO)O-.

15. The method according to claim 13 or 14 wherein the complexing agent is selected from arginine and lauryliminodipropionate.

16. A phosphate derivative of atorvastatin or a derivative thereof obtainable by the method according to any one of claims 5 to 15.

17. A phosphate derivative selected from the group consisting of:
[R-(R*,R*)]-2-(4-fluorophenyl)-β-phosphono-δ-hydroxy-5-(1-methylethyl)-3-phenyl-4-[(phenylamino)carbonyl]-1H-pyrrale-1-heptanoic acid; and
1,2-distearoyl phosphatidyl atorvastatin.

18. A phosphate derivative according to any one of claims 1 to 4, 16 or 17 for use in lowering patient serum cholesterol levels.

19. A phosphate derivative according to any one of claims 1 to 4, 16 or 17 for use in treating depression.

20. Use of a phosphate derivative according to any one of claims 1 to 4, 16 or 17 for lowering patient serum cholesterol levels.

21. Use of a phosphate derivative according to any one of claims 1 to 4, 16 or 17 for treating depression.

22. The phosphate derivative according to claim 18 or 19 or the use according to claim 20 or 21 wherein the compound is atorvastatin.
